# EUROPÄISCHE PATENTSCHRIFT

(11) **EP 2 699 544 B1**
(45) Veröffentlichungstag und Bekanntmachung des Hinweises auf die Patenterteilung: **08.04.2015**
(21) Anmeldenummer: 12715388.0
(22) Anmeldetag: 19.04.2012
(51) Int. Cl.: C07C 319/28, C07C 323/58

(54) **VERFAHREN ZUR REINIGUNG VON L-CYSTEIN**
METHOD FOR PURIFYING L-CYSTEINE
PROCÉDÉ DE PURIFICATION DE L-CYSTÉINE

(30) Priorität: 20.04.2011 DE 102011007790
(43) Veröffentlichungstag der Anmeldung: 26.02.2014
(73) Patentinhaber: Wacker Chemie AG, 81737 München (DE)
(72) Erfinder: LEUTE, Maria, 81475 München (DE); BÖHM, Andreas, 81829 München (DE)
(74) Vertreter: Potten, Holger
(86) Internationale Anmeldenummer: PCT/EP2012/057109
(87) Internationale Veröffentlichungsnummer: WO 2012/143412

(56) Entgegenhaltungen:
- EP-A1- 1 958 933

## Beschreibung

Die Erfindung betrifft ein Verfahren zur Reinigung von L-Cystein aus einer L-Cystein-haltigen Fermenterbrühe.

L-Cystein ist eine Aminosäure, die aufgrund der guten Löslichkeit in Wasser und der hohen Empfindlichkeit der SH-Gruppe gegenüber einer Vielzahl von Reagenzien, z.B. gegenüber Oxidationsmitteln, nur sehr schwer und unter hohem Aufwand zu reinigen und zu isolieren ist.

US2590209 beschreibt ein allgemeines Verfahren, Aminosäure-Gemische in saure, neutrale und basische Aminosäuren aufzutrennen, bei dem ein saures Proteinhydrolysat zunächst über einen Kationenaustauscher geleitet wird, dann die sauren und neutralen Aminosäuren mit Ammoniumhydroxid eluiert werden, während die basischen Aminosäuren zurückgehalten werden. Diese werden im Anschluss mit einer Carbonatlösung vom Kationenaustauscher gespült. Die sauren und neutralen Aminosäuren wiederum werden mittels nachgeschaltetem Anionenaustauschers getrennt, an welchem nur die sauren Aminosäuren gebunden werden. Das beschriebene Verfahren ist nicht für die Aufreinigung von Cystein geeignet, da Basen als Elutionsmittel eingesetzt werden, Cystein jedoch bei hohen pH-Werten besonders oxidationsempfindlich ist.

Probleme bei der Aufreinigung sowie der Stand der Technik zur Gewinnung von L-Cystein sind in EP 1958933 A1 (entspricht US 2008-0190854) beschrieben. Diese Anmeldung beschreibt ein Verfahren, bei dem eine L-Cystein-haltige Fermenterbrühe, die ein Oxidationsmittel umfasst, welches bei pH-Werten von pH<5 L-Cystein zu oxidieren vermag, bei einem pH Wert von pH 5 bis 9 mit einem Ionenaustauscher in Kontakt gebracht wird wobei ein pH-Wert von pH<5, bevorzugt pH<2 in der Fermenterbrühe auftritt. Dabei bindet das L-Cystein an den Ionenaustauscher und das gebundene L-Cystein wird mittels eines Elutionsmittels vom Ionenaustauscher entfernt. Aus dem Eluat kann anschließend L-Cystein-Hydrochlorid-Monohydrat durch fraktionierte Kristallisation gewonnen werden. Zunächst werden durch Zugabe von konzentrierter HCl oder durch Einleiten von HCl-Gas Chloride anorganischer Alkali- und Erdalkalimetallionen sowie Ammoniumchlorid kristallisiert. Nach Filtration wird die L-Cystein enthaltende Mutterlauge auf -10°C gekühlt und L-Cystein-Hydrochlorid-Monohydrat kristallisiert. Ausbeute und Reinheit sind jedoch von der Reinheit der eingesetzten L-Cystein enthaltenden Lösung abhängig, Reinheiten bis zu >98 Gew. % erfordern weitere fraktionierte Kristallisation und/oder Umkristallisation.

Verfahren zur fermentativen Herstellung von L-Cystein sind beispielsweise aus EP0885962B1 (entspricht US5972663A) bekannt. Diese Verfahren ermöglichen einen kostengünstigen Zugang zu Fermentationsbrühen, die L-Cystein in großer Menge enthalten.

Eine derartige L-Cystein enthaltende Fermenterbrühe ist ein komplexes Substanzgemisch. Sie enthält neben L-Cystein in der Regel L-Cystin, welches unter den Bedingungen der Fermentation, insbesondere durch Oxidation mit vorhandenem Sauerstoff, leicht aus L-Cystein gebildet wird. Bei Anwesenheit von Aldehyden oder Ketonen können des Weiteren entsprechende Hemithioketale und/oder Thiazolidinderivate von L-Cystein, wie z.B. in EP0885962B1 beschrieben, vorliegen. In geringen Mengen können die Fermenterbrühen auch weitere Aminosäuren oder deren Derivate enthalten. Sie enthalten ferner in der Regel Kohlenhydrate, Salze organischer und anorganischer Kationen und Anionen, z.B. Alkali- und Erdalkalimetallsalze sowie Spuren an Schwermetallsalzen (z.B. Fe, Cu, Mn, Zn etc.), Farbstoffe und weitere Verunreinigungen und Zusätze, wie z.B. unerwünschte Stoffwechselprodukte der bei der Fermentation eingesetzten Mikroorganismen. Ferner können die Fermenterbrühen noch die in der Fermentation eingesetzten Roh- und Inhaltsstoffe enthalten, z.B. übliche C-Quellen, wie Glucose, Lactose, Stärke und dergleichen, N-Quellen, wie Ammoniak/Ammonium oder Proteine oder Proteinhydrolysate und dergleichen, sowie S-Quellen, wie beispielsweise Sulfid, Sulfit, Sulfat, Thiosulfat oder Dithionit und dergleichen. Da L-Cystein eine schwefelhaltige Aminosäure ist, wird während der Fermentation in aller Regel eine S-Quelle, wie beispielsweise Sulfid, Sulfit, Sulfat, Thiosulfat oder Dithionit zugefüttert, um den für die Bildung von L-Cystein benötigten Schwefel in ausreichender Menge bereitzustellen. Weiterhin ist in den Fermenterbrühen durch den während der Fermentation eingetragenen Sauerstoff auch gelöster Sauerstoff enthalten. Der pH-Wert dieser Fermenterbrühen liegt üblicherweise, wie z.B. in EP0885962B1 beschrieben, bei pH 7. Neben der Komplexität der Zusammensetzung der Fermenterbrühe, treten zusätzlich natürliche Schwankungen im Verhältnis der einzelnen Inhaltsstoffe auf, da es sich um ein Produkt biologischer Prozesse handelt.

Aufgabe der vorliegenden Erfindung ist es, ein einfaches, kostengünstiges und technisch durchführbares Verfahren zur Herstellung einer gereinigten L-Cystein-enthaltenden Lösung, aus einer L-Cystein-haltigen Fermenterbrühe bereitzustellen.

Diese Aufgabe wird gelöst durch ein Verfahren, bei dem eine L-Cystein-haltige Fermenterbrühe bei einem pH Wert von pH 6 bis 9 mit einem basischen Anionenaustauscher in Kontakt gebracht wird, wobei das L-Cystein an den Anionenaustauscher bindet, der Anionenaustauscher mit einer ersten Waschlösung gespült wird, das gebundene L-Cystein mittels einer Säure vom Anionenaustauscher entfernt und in ein Eluat überführt wird und das Eluat mit einem pH ≤ 4 mit einem sauren Kationenaustauscher in Kontakt gebracht wird, wobei das L-Cystein an den Kationenaustauscher bindet, der Kationenaustauscher mit einer zweiten Waschlösung gespült wird, und das gebundene L-Cystein mittels einer starken Säure vom Kationenaustauscher entfernt wird.

Das erfindungsgemäße Verfahren liefert unabhängig von Schwankungen in der Zusammensetzung der Fermenterbrühe stets eine einheitliche Lösung, da L-Cystein zuverlässig und nahezu vollständig von allen Begleitsubstanzen abgetrennt wird. Aus dieser Lösung kann dann bei Bedarf L-Cystein, L-Cystein-Hydrochlorid oder L-Cystein-Hydrochlorid-Monohydrat als Feststoff, gewonnen werden. Vorzugsweise wird die erhaltene Lösung dazu aufkonzentriert bis das gewünschte Produkt, bevorzugt L-Cystein-Hydrochlorid-Monohydrat auskristallisiert. Das so erhaltene L-Cystein-Hydrochlorid-Monohydrat weist eine Reinheit >98% Gew.% auf. Eine fraktionierte Kristallisation wie in EP1958933A1 beschrieben ist nicht notwendig.

Eine L-Cystein enthaltende Fermenterbrühe wird, vorzugsweise nach Abtrennung der Zellen und Feststoffe, mit Hilfe des erfindungsgemäßen Verfahrens zunächst mittels eines basischen Ionenaustauschers und anschließend mittels eines sauren Ionenaustauschers aufgereinigt.

Saure und basische Ionenaustauscher sind bekannt und kommerziell verfügbar. Eine Auswahl verschiedener geeigneter Materialen ist in Ullmann's Encyclopedia of Industrial Chemistry, Vol. A14, S. 451 zusammengestellt. Als aktive ionenaustauschende Gruppen enthalten sie beispielsweise Carbonsäuregruppen (schwach saure Ionenaustauscher), Sulfonsäure und Phosphonsäuregruppen (stark saure Ionenaustauscher), quartäre Ammoniumgruppen (stark basische Ionenaustauscher) oder Amingruppen (schwach basische Ionenaustauscher). Als Gegenionen zu den aktiven ionenaustauschenden Gruppen können Kationen oder Anionen an den Ionenaustauscher gebunden sein. Saure Ionenaustauscher werden häufig in der protonierten H⁺-Form eingesetzt, gängige weitere Gegenionen sind aber z.B. auch Ammoniumionen, Alkali- und/oder Erdalkalimetallionen. Basische, insbesondere stark basische Ionenaustauscher, werden häufig in der OH⁻-Form eingesetzt, gängige weitere Gegenionen sind aber z.B. auch Chlorid und weitere im Stand der Technik beschriebene Anionen.

Bevorzugt werden stark saure und stark basische Ionenaustauscher eingesetzt. Der Anionenaustauscher wird bevorzugt in der OH⁻-Form eingesetzt, der Kationenaustauscher bevorzugt in der H⁺-Form. Es ist aber prinzipiell auch möglich Ionenaustauscher mit anderen Gegenionen zu verwenden.

Vorzugsweise wird die L-Cystein enthaltende Fermenterbrühe zuerst mit einem stark basischen Anionenaustauscher in Kontakt gebracht, und das L-Cystein-haltige Eluat des Anionenaustauschers anschließend mit einem stark sauren Kationenaustauscher in Kontakt gebracht. Dies kann z.B. dadurch geschehen, dass Fermenterbrühe bzw. Eluat über mit den entsprechenden Ionenaustauschern gepackte Säulen gepumpt wird. Der pH-Wert der applizierten Fermenterbrühe entspricht dem pH-Wert während der Fermentation und liegt in einem pH-Bereich von 6-9 und bevorzugt bei einem pH von 6-8.

Das L-Cystein enthaltende Eluat des Anionenaustauschers wird bevorzugt mit einem pH-Wert von 1 bis 4 auf den Kationenaustauscher appliziert.

Bei der ersten Waschlösung handelt es sich vorzugsweise um Wasser mit einem pH-Wert im Bereich von 6 bis 8, besonders bevorzugt um Wasser mit einem pH-Wert von 7 (neutrales Wasser). Durch das Waschen mit dieser Waschlösung werden neutrale und kationische Bestandteile der Fermenterbrühe vom Anionenaustauscher entfernt.

Bei der zweiten Waschlösung handelt es sich vorzugsweise um Wasser mit einem pH-Wert im Bereich von 4 bis 8, besonders bevorzugt um Wasser mit einem pH-Wert im Bereich 5 bis 7. Durch das Waschen mit neutralem bis leicht saurem Wasser werden anionische Verunreinigungen vom Kationenaustauscher entfernt.

Alle Angaben zum pH-Wert im Rahmen der vorliegenden Anmeldung beziehen sich auf eine Bestimmung des pH-Wertes bei 25°C.

Im Rahmen der Versuche die zur vorliegenden Erfindung führten zeigte sich überraschend, dass L-Cystein, welches bei pH-Werten >7 leicht durch Luftsauerstoff oder andere Oxidationsmittel zu L-Cystin umgesetzt wird, ohne nennenswerte Oxidation an einem basischen Ionenaustauscher adsorbiert und von diesem wieder eluiert werden kann. Dies war deshalb besonders überraschend, da eine deutliche Oxidation von L-Cystein zu L-Cystin beobachtet wird, wenn beim Eluieren von L-Cystein, welches an einem sauren Kationenaustauscher gebunden ist, eine Base, z.B. wässriger Ammoniak, verwendet wird. Durch diese Oxidation tritt ein unmittelbarer deutlicher Ausbeuteverlust auf (siehe Beispiele 3 und 6), durch den die Wirtschaftlichkeit des Verfahrens nicht mehr gegeben ist.

Wird eine neutrale L-Cystein enthaltende Lösung bzw. Fermenterbrühe mit einem Anionenaustauscher, bevorzugt einem stark basischen Anionenaustauscher in der OH⁻-Form, in Kontakt gebracht, so wird bei diesem Vorgang L-Cystein nahezu quantitativ an den Anionenaustauscher gebunden. Auch andere Aminosäuren, z.B. L-Cystin, und/oder weitere Anionen, welche in der Fermenterbrühe enthalten sein können, können hierbei an das Ionenaustauscherharz binden. Eine Vielzahl von weiteren Verunreinigungen, wie z.B. Neutralverbindungen oder Kationen bzw. deren korrespondierende Basen, bindet nicht an das Anionenaustauscherharz und befindet sich im Ablauf.

Nach Elution mit einer Säure wird die L-Cystein enthaltende Lösung mit einem Kationenaustauscher, bevorzugt einem stark sauren Kationenaustauscher in der H⁺-Form, in Kontakt gebracht, dabei wird L-Cystein quantitativ an den Kationenaustauscher gebunden. Noch enthaltene anionische Verunreinigungen binden nicht an das Kationenaustauscherharz und befinden sich im Ablauf.

Als Elutionsmittel werden bevorzugt starke Säuren worunter vorzugsweise Säuren mit einem pKs Wert kleiner 4,5 zu verstehen sind, und besonders bevorzugt Salzsäure eingesetzt. Insbesondere geeignet ist wässrige Salzsäure.

Bei Elution mit Salzsäure wird durch den mit L-Cystein beladenen Anionenaustauscher wässrige Salzsäure vorzugsweise unterschiedlicher Normalitäten, bevorzugt 0,01-12 N HCl und besonders bevorzugt 0,01-1N HCl gepumpt. Insbesondere bevorzugt wird ein Elutionsmittel in dem die HCl-Konzentration während der Elution von anfangs 0,01 N auf bis zu 1 N gesteigert wird.

Bei Elution mit Salzsäure wird durch den mit L-Cystein beladenen Kationenaustauscher vorzugsweise wässrige Salzsäure unterschiedlicher Normalitäten, bevorzugt 0,1-12N HCl und besonders bevorzugt 1-2N HCl gepumpt.

Beladung, Waschschritt und Elution werden bevorzugt bei 25°C und beim Druck der umgebenden Atmosphäre, also 900 bis 1100 hPa, durchgeführt. Er kann aber auch bei niedrigeren oder höheren Temperaturen und Drücken durchgeführt werden.

Bei Elution des L-Cysteins mit Salzsäure wird eine gereinigte HCl-Lösung von L-Cystein erhalten. Diese Lösung kann optional aufkonzentriert und z.B. mit Aktivkohle entfärbt werden. Ggf. nach Zugabe von HCl wird das technisch besonders wichtige Produkt L-Cystein-Hydrochlorid-Monohydrat kristallisiert. Durch die Aufreinigung mittels doppelten Ionenaustauschers kann L-Cystein in hoher Reinheit, unabhängig von der Zusammensetzung der ursprünglich applizierten Fermenterbrühe, erhalten werden. Eine weitere Aufreinigung z.B. durch fraktionierte Kristallisation ist nicht notwendig.

Mit dem erfindungsgemäßen Verfahren ist es möglich L-Cystein effektiv, mit guten Ausbeuten und wirtschaftlich aus einer L-Cystein enthaltenden Fermenterbrühen aufzureinigen. Wenn es erforderlich oder gewünscht ist, können unter bestimmten Verfahrensbedingungen z.B. auch evtl. vorhandene Derivate von L-Cystein, z.B. L-Cystin oder Thiazolidin-Derivate, zu L-Cystein umgewandelt werden und somit die erzielte Ausbeute erhöhen. So ist z.B. die Spaltung von Thiazolidin-Derivaten des Cysteins an stark sauren Kationenaustauschern bekannt und in EP 1059288 B1 beschrieben. Die Spaltung von L-Cystin zu L-Cystein durch Zugabe von geeigneten Reduktionsmitteln ist ebenfalls denkbar.

Mit dem beschriebenen Verfahren ist es möglich unabhängig von der Zusammensetzung der ursprünglich applizierten Fermenterbrühe den Fremdaminosäure-Gehalt auf <5 Gew. %, bevorzugt <1 Gew. % bezogen auf L-Cystein zu senken. Des Weiteren ist es auch möglich den Salzgehalt bezogen auf L-Cystein auf <10 Gew. %, bevorzugt <1 Gew. % zu senken. Außerdem ist es mit dem beschriebenen Verfahren möglich L-Cystein bzw. L-Cystein-Hydrochlorid oder L-Cystein-Hydrochlorid-Monohydrat in einer Reinheit von >98 Gew. % und einer optischen Reinheit von >99 % ohne zusätzliche Aufreinigung z.B. durch fraktionierte Kristallisation, aus L-Cystein enthaltenden Fermenterbrühen herzustellen.

Bevorzugt erfolgt vor Durchführung des erfindungsgemäßen Verfahrens in einem ersten Verfahrensschritt die Abtrennung von Mikroorganismenzellen und/oder unlösliche Bestandteilen aus der L-Cystein enthaltenden Fermenterbrühe. Dies geschieht beispielsweise durch Zentrifugation, Filtration, Dekantieren, Membranfiltration oder eine andere dem Fachmann geläufigen Methode zur Abtrennung von Zellen/Feststoffen aus einer Fermenterbrühe. Die Abtrennung erfolgt ggf. unter Zusatz eines Filterhilfsmittels wie z.B. Celite^{®}, Aktivkohle oder Diatomeenerde. Bei diesem Verfahrensschritt werden vorteilhafterweise neben den Zellen weitere unlösliche Bestandteile, wie z.B. aus der Lösung ausgefallenes Cystin oder Niederschläge anderer schwerlöslicher Bestandteile, die bei der Fermentation des Mikroorganismus entstehen können, ebenfalls von der Fermenterbrühe abgetrennt. Des Weiteren können bei diesem Verfahrensschritt z.B. auch Makromoleküle, wie z.B. Proteine, abgetrennt bzw. an ein ggf. eingesetztes Filterhilfsmittel oder Aktivkohle oder Ähnlichem adsorbiert und somit abgetrennt werden. Die mittels dieser Vorbehandlung erhaltene L-Cystein-haltige Lösung fällt im Sinne der vorliegenden Erfindung auch unter den Begriff der Fermenterbrühe.

Die folgenden Beispiele dienen der weiteren Erläuterung der Erfindung.

### Beispiel 1: Aufreinigung einer L-Cystein-haltigen Fermenterbrühe mittels des erfindungsgemäßen Verfahrens

500 ml zellfreie Fermenterbrühe (24 g/l L-Cystein, 1,2 g/l L-Cystin, 18 g/l N-Acetylserin) wurden mit nativem pH (pH: 7) auf 150 ml eines Anionenaustauscherharzes (OH⁻-Form), käuflich erhältlich unter der Bezeichnung Amberlite^{®} FPA 42Cl bei Rohm & Haas aufgetragen. Das Harz wurde dann mit 250 ml Wasser (pH 7) gewaschen und mit 1750 ml HCl eluiert. Dabei wurde die Säurekonzentration alle 250 ml schrittweise erhöht, von 0,01 M HCl zu 0,1 M HCl zu 0,25 M HCl zu 0,5 M HCl.
Der Cystein-Gehalt der Eluat-Fraktionen wurde jeweils mittels HPLC bestimmt und die Cystein-haltigen Fraktionen vereinigt. Davon 500 ml wurden auf 120 ml eines Kationenaustauscherharzes (H⁺-Form), käuflich erhältlich unter der Bezeichnung Amberlite^{®} FPC 14-Na bei Rohm & Haas, aufgetragen, Das Harz wurde dann mit 120 ml Wasser gewaschen und mit 720 ml 1 M HCl eluiert.

Die Cystein-haltigen Fraktionen im Eluat des Anionenaustauschers enthielten mehr als 85 % des in der applizierten Fermenterbrühe vorhandenen L-Cysteins, die Ausbeute an L-Cystein in den Produktfraktionen im Eluat des Kationenaustauscherharzes war quantitativ.

### Beispiel 2: Vergleichsbeispiel: Aufreinigung einer L-Cystein-haltigen Fermenterbrühe ausschließlich mittels Kationenaustauscher (entspricht Verfahren in EP 1958933 A1), Elutionsmittel: HCl

6500 ml einer zellfreie Fermenterbrühe mit einem pH-Wert von pH = 7, wurden mit 6 M HCl auf pH = 1 angesäuert und zur Abtrennung denaturierter Proteine und sonstiger Feststoffen nach 1 h zentrifugiert. 5350 ml der klaren, salzsauren Lösung ((19 g/l L-Cystein, 1,4 g/l L-Cystin, 8 g/l N-Acetylserin)) wurden mittels Kationenaustauscher (1100 ml Amberlite^{®} FPC 14-Na, H⁺-Form) aufgereinigt. Die Lösung wurde auf den Ionenaustauscher aufgetragen, die Säule mit 3300 ml Wasser gewaschen und mit 5500 ml 1 M HCl eluiert. Die Cystein-haltigen Fraktionen des Eluats enthielten 85 % der eingesetzten Cysteinmenge, sowie in der Lösung vorhandene Kationen.

### Beispiel 3: Vergleichsbeispiel: Aufreinigung einer L-Cystein-haltigen Fermenterbrühe unter basischen Bedingungen, mittels Kationenaustauscher, Elutionsmittel: NH₄OH

100 ml einer zellfreie Fermenterbrühe mit einem pH-Wert von pH = 7, wurden mit 6 M HCl auf pH = 1 angesäuert und zur Abtrennung denaturierter Proteine und sonstiger Feststoffen nach 1 h zentrifugiert. Die klare, salzsaure Lösung ((20 g/l L-Cystein, 1,2 g/l L-Cystin, 8 g/l N-Acetylserin)) wurden mittels Kationenaustauscher (20 ml Amberlite^{®} FPC 14-Na, H⁺-Form) aufgereinigt. Die Lösung wurde auf den Ionenaustauscher aufgetragen, die Säule mit 60 ml Wasser gewaschen und mit 100 ml 2 M NH₃ eluiert. Die Produkt-Fraktionen enthalten ca. 50 % des in der applizierten Fermenterbrühe vorhandenen L-Cysteins, sowie gegenüber der Ausgangsmenge deutlich erhöhte Cystinmengen.

### Beispiel 4: Kristallisation von L-Cystein-Hydrochlorid-Monohydrat aus den HCl-Eluaten aus Bsp. 1

500 ml einer gereinigten L-Cystein enthaltenden Lösung (Eluat mit 1 M HCl) aus Beispiel 1 wurden bei 45°C und 50 mbar einrotiert bis zum Beginn der Kristallisation, dann wurde die Lösung langsam auf Raumtemperatur gebracht, und anschließend wurde die Temperatur kontinuierlich gesenkt, bis auf -18°C. Nach 3h Rührzeit wurde der Niederschlag mit einer vorgekühlten Nutsche abgesaugt und an der Luft getrocknet.
Ausbeute: 6,5 g feinkristalliner Feststoff, >98 Gew. % Cys-tein-Hydrochlorid-Monohydrat (entspricht 70 % Cystein-Gesamtausbeute bezogen auf Ausgangslösung = zellfreie Fermenterbrühe)

### Beispiel 5: Vergleichsbeispiel: Kristallisation von L-Cystein-Hydrochlorid-Monohydrat aus den HCl-Eluaten aus Bsp. 2

5500 ml einer gereinigten L-Cystein enthaltenden Lösung (Eluat mit 1M HCl) aus Beispiel 2 wurden bei 45°C und 50 mbar einrotiert bis zum Beginn der Kristallisation, dann wurde die Lösung langsam auf Raumtemperatur gebracht, und anschließend wurde die Temperatur kontinuierlich gesenkt, bis auf -18°C. Nach 3h Rührzeit wurde der Niederschlag mit einer vorgekühlten Nutsche abgesaugt und an der Luft getrocknet.
Ausbeute: 120 g feinkristalliner Feststoff, 79 wt% Cystein-Hydrochlorid-Monohydrat (entspricht 64 % Cystein-Gesamtausbeute bezogen auf Ausgangslösung = zellfreie Fermenterbrühe)

### Beispiel 6: Vergleichsbeispiel: Kristallisation von L-Cystein aus den NH₃-Eluaten aus Bsp. 3

100 ml einer gereinigten L-Cystein enthaltenden Lösung (Eluat mit 2 M NH₃) aus Beispiel 3 wurden bei 45°C und 50 mbar einrotiert bis zum Beginn der Kristallisation, dann wurde die Lösung langsam auf Raumtemperatur gebracht, und anschließend wurde die Temperatur kontinuierlich gesenkt, bis auf -18°C. Nach 3h Rührzeit wurde der Niederschlag mit einer vorgekühlten Nutsche abgesaugt und an der Luft getrocknet.
Ausbeute: die Analyse des erhaltenen Produkts zeigte, dass ca. 90% des eingesetzten L-Cysteins unter diesen Bedingungen zu L-Cystin umgesetzt wurden.

## Patentansprüche

1. Verfahren zur Herstellung einer gereinigten L-Cystein-enthaltenden Lösung aus einer L-Cystein-haltige Fermenterbrühe **dadurch gekennzeichnet, dass** die L-Cystein-haltige Fermenterbrühe bei einem pH Wert von pH 6 bis 9 mit einem basischen Anionenaustauscher in Kontakt gebracht wird, wobei das L-Cystein an den Anionenaustauscher bindet, der Anionenaustauscher mit einer ersten Waschlösung gespült wird, das gebundene L-Cystein mittels einer Säure vom Anionenaustauscher entfernt und in ein Eluat übergeführt wird und das Eluat mit einem pH ≤ 4 mit einem sauren Kationenaustauscher in Kontakt gebracht wird wobei das L-Cystein an den Kationenaustauscher bindet, der Kationenaustauscher mit einer zweiten Waschlösung gespült wird, und das gebundene L-Cystein mittels einer starken Säure vom Kationenaustauscher entfernt wird.

2. Verfahren gemäß Anspruch 1 **dadurch gekennzeichnet, dass** stark saure und stark basische Ionenaustauscher eingesetzt werden.

3. Verfahren gemäß Anspruch 1 oder 2 **dadurch gekennzeichnet, dass** der Anionenaustauscher in der OH⁻-Form und der Kationenaustauscher in der H⁺-Form eingesetzt wird.

4. Verfahren gemäß einem der Ansprüche 1 bis 3, **dadurch gekennzeichnet, dass** es sich bei der ersten Waschlösung um Wasser mit einem pH-Wert im Bereich von 6 bis 8, besonders bevorzugt um Wasser mit einem pH-Wert von 7 handelt.

5. Verfahren gemäß einem der Ansprüche 1 bis 4, **dadurch gekennzeichnet, dass** es sich bei der zweiten Waschlösung um Wasser mit einem pH-Wert im Bereich von 4 bis 8, besonders bevorzugt um Wasser mit einem pH-Wert im Bereich von 5 bis 7 handelt.

6. Verfahren nach einem der Ansprüche 1 bis 5, **dadurch gekennzeichnet, dass** von der Fermenterbrühe zunächst Zellen und Feststoffe abgetrennt werden.

7. Verfahren nach einem der Ansprüche 1 bis 6, **dadurch gekennzeichnet, dass** die erhaltene Lösung zunächst aufkonzentriert und anschließend aus der aufkonzentrierten Lösung L-Cystein-Hydrochlorid-Monohydrat auskristallisiert.

8. Verfahren gemäß Anspruch 7 **dadurch gekennzeichnet, dass** das erhaltene L-Cystein-Hydrochlorid-Monohydrat eine Reinheit >98 Gew. % aufweist.

## Claims

1. Process for preparing a purified L-cysteine-containing solution from an L-cysteine-containing fermentation broth, **characterized in that** the L-cysteine-containing fermentation broth is brought into contact at a pH of from 6 to 9 with a basic anion exchanger, where the L-cysteine binds to the anion exchanger, the anion exchanger is flushed with a first washing solution, the bound L-cysteine is removed from the anion exchanger by means of an acid and transferred into an eluate and the eluate is brought into contact at a pH of ≤ 4 with an acidic cation exchanger, where the L-cysteine binds to the cation exchanger, the cation exchanger is flushed with a second washing solution and the bound L-cysteine is removed from the cation exchanger by means of a strong acid.

2. Process according to Claim 1, **characterized in that** strongly acidic and strongly basic ion exchangers are used.

3. Process according to Claim 1 or 2, **characterized in that** the anion exchanger is used in the OH⁻ form and the cation exchanger is used in the H⁺ form.

4. Process according to any of Claims 1 to 3, **characterized in that** the first washing solution is water having a pH in the range from 6 to 8, particularly preferably water having a pH of 7.

5. Process according to any of Claims 1 to 4, **characterized in that** the second washing solution is water having a pH in the range from 4 to 8, particularly preferably water having a pH in the range from 5 to 7.

6. Process according to any of Claims 1 to 5, **characterized in that** cells and solids are firstly separated off from the fermentation broth.

7. Process according to any of Claims 1 to 6, **characterized in that** the solution obtained is firstly concentrated and L-cysteine hydrochloride monohydrate is subsequently crystallized out from the concentrated solution.

8. Process according to Claim 7, **characterized in that** the L-cysteine hydrochloride monohydrate obtained has a purity of >98% by weight.

## Revendications

1. Procédé pour la préparation d'une solution purifiée contenant de la L-cystéine à partir d'un bouillon de fermenteur contenant de la L-cystéine, **caractérisé en ce que** le bouillon de fermenteur contenant de la L-cystéine est mis en contact à un pH de 6 à 9 avec un échangeur d'anions basique, la L-cystéine se liant à l'échangeur d'anions, l'échangeur d'anions est rincé avec une première solution de lavage, la L-cystéine liée est éliminée de l'échangeur d'anions à l'aide d'un acide et transférée dans un éluat et l'éluat présentant un pH ≤ 4 est mis en contact avec un échangeur de cations acide, la L-cystéine se liant à l'échangeur de cations, l'échangeur de cations est rincé avec une deuxième solution de lavage et la L-cystéine liée est éliminée au moyen d'un acide fort de l'échangeur de cations.

2. Procédé selon la revendication 1, **caractérisé en ce qu'**on utilise des échangeurs d'ions fortement acides et fortement basiques.

3. Procédé selon la revendication 1 ou 2, **caractérisé en ce que** l'échangeur d'anions est utilisé sous forme OH⁻ et l'échangeur de cations est utilisé sous forme H⁺.

4. Procédé selon l'une quelconque des revendications 1 à 3, **caractérisé en ce qu'**il s'agit, pour la première solution de lavage, d'eau présentant un pH dans la plage de 6 à 8, de manière particulièrement préférée d'eau présentant un pH de 7.

5. Procédé selon l'une quelconque des revendications 1 à 4, **caractérisé en ce qu'**il s'agit, pour la deuxième solution de lavage, d'eau présentant un pH dans la plage de 4 à 8, de manière particulièrement préférée d'eau présentant un pH dans la plage de 5 à 7.

6. Procédé selon l'une quelconque des revendications 1 à 5, **caractérisé en ce que** les cellules et les solides sont d'abord éliminés du bouillon de fermenteur.

7. Procédé selon l'une quelconque des revendications 1 à 6, **caractérisé en ce que** la solution obtenue est d'abord concentrée, puis du chlorhydrate de L-cystéine monohydraté est séparé par cristallisation de la solution concentrée.

8. Procédé selon la revendication 7, **caractérisé en ce que** le chlorhydrate de L-cystéine monohydraté obtenu présente une pureté > 98% en poids.
